# EUROPEAN PATENT SPECIFICATION

(11) **EP 2 268 187 B1**
(45) Date of publication and mention of the grant of the patent: **14.09.2016**
(21) Application number: 09725659.8
(22) Date of filing: 23.03.2009
(51) Int. Cl.: A61B 1/005, A61B 1/04, A61B 1/05

(54) **FLEXIBLE ENDOSCOPE WITH CORE MEMBER**
FLEXIBLES ENDOSKOP MIT KERNELEMENT
ENDOSCOPE FLEXIBLE À ÉLÉMENT DE C UR

(30) Priority: 24.03.2008 US 38872
(43) Date of publication of application: 05.01.2011
(73) Proprietor: Boston Scientific Scimed, Inc., Maple Grove, MN 55311-1566 (US)
(72) Inventor: OSTROVSKY, Isaac, Wellesley MA 02482 (US); MCINTYRE, Jon, T., Newton MA 02465 (US); FAIRNENY, Ty, Hopkinton MA 01748 (US); INTOCCIA, Alfred, Nashua NH 03062 (US); HIXON, Jessica, Watertown MA 02472 (US)
(74) Representative: Pfenning, Meinig & Partner mbB
(86) International application number: PCT/US2009/037937
(87) International publication number: WO 2009/120622

(56) References cited:
- JP-A- 9 154 807
- JP-A- 10 118 011
- US-A1- 2006 009 759
- US-A1- 2007 043 261

## Description

### CROSS-REFERENCE TO RELATED CASES

This application claims priority to, and the benefit of Provisional U.S. Patent Application Serial No. 61/038,872, filed March 24, 2008, the entirety of which is incorporated herein by reference.

### TECHNICAL FIELD

The present invention generally relates to endoscopes for use during a medical procedure.

### BACKGROUND INFORMATION

Medical devices are used to access regions of the body to deliver diagnostic or therapeutic agents to those regions and to perform surgical procedures on those regions. For example, endoscopes may use body airways and canals to access the colon, esophagus, stomach, urethra, bladder, ureter, kidneys, lungs, bronchi, or uterus. Catheters may use the circulatory system as pathways to access treatment sites near the heart or may use the urinary canal to access urinary regions.

Medical devices are often introduced into the body through a large artery such as those found in the groin or in the neck. The devices are often passed through ever-narrower arteries until they can reach the operative site inside the body. Many such pathways may curve, loop around, and even wind back. In order to navigate the device through the pathways to the operative site, the device must be flexible to allow bending, yet have enough column strength to prevent buckling as the device is pushed.

JP H10 118011 A is directed to an endoscope system using stylet. According to the abstract of JP H10 118011 A, the problem to be solved by the invention described in JP H10 118011 A is to select degree of rigidity and straightness of an inserting part of an endoscope and to insert the endoscope to the deep part by passing an appropriately selected stylet from a plurality of different kinds of the stylets through an through-channel of a processing tool of the endoscope and adjusting the inserting part of the endoscope to a required rigidity and straightness. This problem is solved as follows. An inserting part is connected with the apex of an operating part of an endoscope through a breakproof member and the inserting part is constituted by connecting successively an apex part, a curved part and a flexible part from the apex. In addition, a processing tool inserting hole for inserting a processing tool and a stylet is provided below the operating part and the processing tool inserting hole is communicated with a processing tool inserting channel arranged in the inserting part communicated with the apex part and a forceps plug is freely removably mounted in the processing tool inserting hole. In addition, the stylet for the endoscope is inserted through the processing tool inserting channel through the processing tool inserting hole to adjust degree of rigidity and straightness of the inserting part of the endoscope.

JP H09 154807 A is directed to an endoscope According to the abstract of JP H09 154807 A, the problem to be solved by the invention described in JP H09 154807 A is to simplify handling, and change flexibility of an inserting part without deteriorating a sucking function by housing a long and narrow rigid member inside an endoscope, and arranging a means to move the rigid member in the shaft direction in the inserting part, in a operation part. This problem is solved as follows. A stylet is a rigid member to give rigidity to an inserting part, and at tip part is composed of a tip soft part, and a hand side part is composed of a rigid part, and a part on the more hand side than it is composed of a rear soft part. A space is formed between the rear end of a first guide tube and the tip of a second guide tube, and a moving means of the stylet is arranged in this part. This moving means is provided with two transfer rollers to sandwich the stylet inserted over into the first and the second guide tubes, and at least one is rotated by a motor connected to control device, and a switch to control this motor is arranged in an operation part.

US 2007/0044261 A1 is directed to an endoscope and a method for inserting endoscope into colon. A first bendable section which can be bent in an arbitrary direction and a second bendable section which can be bent in the bending direction of this first bendable section are provided at the tip end side of an insertion portion. A balloon which is inflated/contracted by feed/discharge of a fluid is provided in this second bendable section so that hardness of this second bendable section can be varied and insertion work into a curved portion is facilitated.

US 2006/0009759 A1 is directed to a loop ablation apparatus. Embodiments of the invention provide an ablation apparatus for ablating target tissue adjacent pulmonary veins of a patient. The ablation apparatus can include a tube capable of being advanced around the pulmonary veins to form a loop. The tube can receive or include electrodes to ablate target tissue. Some embodiments provide a loop ablation device, which may include a cannula and two or more electrode rods carrying two or more bipolar electrodes. The electrode rods can be advanced through the distal ends toward the proximal ends of the loop and toward the target tissue. The bipolar electrodes can receive energy to ablate the target tissue. The bipolar electrodes may be surrounded by the liquid within the cannula while ablating the target tissue, The loop ablation device can further include a rotating grasping mechanism coupled to the electrode rods.

### SUMMERY OF THE INTENTION

In one type of endoscope, the image is transmitted by an imaging fiber bundle, which occupies the central, or near central, space of the endoscope shaft. In another type of endoscope, an electrical signal is transmitted from a small camera (such as a CCD or other image sensor) at or near the tip of the endoscope to a display unit, where the signal the signal gets converted into an image for display to an operator of the endoscope. The one or more wires for transmitting the signal occupy less space in the shaft than a fiber bundle occupies in a more traditional endoscope that does not employ electronic imaging.

The present invention generally relates to an endoscope with an elongated shaft with a core member for imparting flexibility and column strength to the shaft. When located at or near the central axis of the endoscope, this allows the core member to add substantially to the column strength of the device while not adding significant bending stiffness due to low moment of inertia. The core member can occupy the space within the shaft that was traditionally occupied by the imaging fiber bundle, when an electronic imaging device is used at or near the end of the shaft of the endoscope and one or wires within the shaft carry signals from the imaging device.

In one aspect, the invention features an endoscope with a handle and an elongated shaft. The handle is at the proximal end of the endoscope and is used to control the endoscope as it is advanced through body pathways to the operative site. The shaft extends distally from the proximal handle. At least part of the shaft, or the entire shaft, comprises an inner member, a core member, and an outer member. The inner member defines at least three lumens. A first lumen is for receiving an instrument. A second lumen is for receiving one or more wires for connecting a camera at a distal portion of the shaft to a display unit. A third lumen is for receiving a core member. The core member is flexible and provides column strength to the shaft. The outer member is disposed over the inner member of the shaft. The outer member defines a lumen through which the inner member extends.

According to one exemplary embodiment of the present invention, an endoscope includes a proximal handle and an elongated shaft extending distally from the proximal handle. At least a portion of the shaft has an inner member defining a first lumen for receiving an instrument, a second lumen for receiving one or more wires for connecting a camera at a distal portion of the elongated shaft to a display unit, and a third lumen. A core member extending through the third lumen of the inner member is flexible and provides column strength to the at least a portion of the shaft. An outer member is disposed over the inner member and defines a lumen through which the inner member extends.

In one aspect, the endoscope of the present invention can be a ureteroscope, a colonoscope, a hysteroscope, a uteroscope, bronchoscope, or a cystoscope. The first lumen can be used for receiving a light-carrying device, a surgical instrument, or a fluid-carrying device. The inner member can be made from a plastic such as silicone or pebax. The outer member can also be made from a plastic material.

In a further aspect of the present invention, the core member of the endoscope further includes a proximal portion and a distal portion coupled to the proximal portion. The proximal portion can be made from a first material and the distal portion can be made from a second material where the first material is less flexible than the second material. The core member can be a hollow tube and can have a circular cross section or a rectangular cross section. The proximal portion has a first diameter or thickness and the distal portion has a second diameter or thickness where the first diameter or thickness is larger than the second diameter or thickness. The distal portion can also be tapered.

In another aspect of the present invention, the core member also includes a transitional portion coupling the proximal portion to the distal portion. The proximal portion can have a circular cross section and the distal portion can have a rectangular cross section and the cross section of the transitional portion progressively changes from circular to rectangular.

In yet a further aspect of the present invention the core member has a circular cross section and the proximal portion has a first diameter and includes a deflection portion having a second diameter that is smaller than the first diameter to promote deflection of the elongated shaft along the deflection portion. The core member can also have a rectangular cross section and the proximal portion has a first thickness and includes a deflection portion having a second thickness that is smaller than the first thickness to promote deflection of the elongated shaft along the deflection portion. The distal portion can also include such a deflection portion having a cross sectional area that is smaller than the distal portion cross sectional area to promote deflection of the elongated shaft along the deflection portion.

### BRIEF DESCRIPTION OF THE DRAWINGS

For a fuller understanding of the nature and operation of various embodiments according to the present invention, reference is made to the following description taken in conjunction with the accompanying drawing figures which are not necessarily to scale and wherein like reference characters denote corresponding or related parts throughout the several views.
FIG. 1 is a diagram of an endoscope with a proximal handle and an elongated shaft extending distally from the proximal handle.
FIG. 2A is a diagram depicting at least a portion of the elongated shaft.
FIG. 2B is a diagram depicting the axial structure of a proximal portion of the elongated shaft.
FIG. 2C is a diagram depicting the axial structure of a distal portion of the elongated shaft.
FIG. 2D is a diagram depicting an embodiment of the elongated shaft in which the core member has a rectangular cross section.
FIG. 3A is a diagram depicting a configuration of the core member with a circular cross section and including a proximal portion and a distal portion, the proximal portion having a larger diameter than the distal portion.
FIG. 3B is a diagram depicting the flexibility of the proximal and distal portions of the core member.
FIG. 3C is a diagram depicting 360 degrees of bendability of the core member with a circular cross section.
FIG. 4A is a diagram depicting a configuration of the core member, wherein the distal portion of the core member has a diameter that tapers from a proximal end to a distal end of the distal portion.
FIG. 4B is a diagram depicting the increasing flexibility of the core member toward the distal end of the distal portion, the distal portion having a tapering diameter.
FIG. 5A is a diagram depicting a configuration of the core member with a rectangular cross section and including a proximal portion and a distal portion, the proximal portion having a larger thickness than the distal portion.
FIG. 5B is a diagram depicting how the core member with a rectangular cross section promotes flexibility in a single plane.
FIG. 6 is a diagram depicting a configuration of the core member, wherein the distal portion of the core member has a thickness that tapers from a proximal end to a distal end of the distal portion.
FIG. 7 is a diagram depicting a configuration of the core member where the core member is a hollow tube and the core member includes a proximal portion with a first diameter and a distal portion with a second diameter, the first diameter being larger than the second diameter.
FIG. 8A is a diagram depicting a configuration of the core member with a proximal portion comprised of a first material and distal portion comprised of a second material, the first material being less flexible than the second material.
FIG. 8B shows an example of how the first material of the core member of FIG. 8A is more flexible than the second material of the core member of FIG. 8A.
FIG. 9A is a diagram depicting a configuration of the core member with a proximal portion, a distal portion, and a transitional portion coupling the proximal and distal portions. The core member has a circular cross section and the proximal portion has a first diameter and the distal portion has a second diameter that is smaller than the first diameter. The transitional portion has a diameter that reduces from the first diameter to the second diameter.
FIG. 9B is a diagram depicting a configuration of the core member with a proximal portion, a distal portion, and a transitional portion coupling the proximal and distal portions. The core member has a rectangular cross section and the proximal portion has a first thickness and the distal portion has a second thickness that is smaller than the first thickness. The transitional portion has a thickness that reduces from the first thickness to the second thickness.
FIG. 10 is a diagram depicting a configuration of the core member, wherein the transitional portion has a cross section that progressively changes from circular to rectangular.
FIG. 11 is a diagram depicting a configuration of the core member, wherein the core member is a hypotube with a proximal portion, a distal portion, and a transitional portion, the transitional portion having a helically cut slot with a first pitch, and the distal portion having a helically cut slot with a second pitch, the first pitch being larger than the second pitch to promote more flexibility in the distal portion than in the transitional and proximal portions.
FIG. 12 is a diagram depicting a configuration of the core member with a circular cross section, wherein the proximal portion has a deflection portion with diameter reduced from the diameter of the proximal portion to promote 360 degrees of flexibility along the deflection portion.
FIG. 13A is a diagram depicting a configuration of the core member with a rectangular cross section, wherein the proximal portion has a deflection portion with thickness reduced from the thickness of the proximal portion to promote a single plane of flexibility along the deflection portion.
FIG. 13B is a diagram depicting the core member of FIG. 13A bending in the single plane of flexibility at the deflection portion, where the thinner side of the core member is in the plane.

### DESCRIPTION

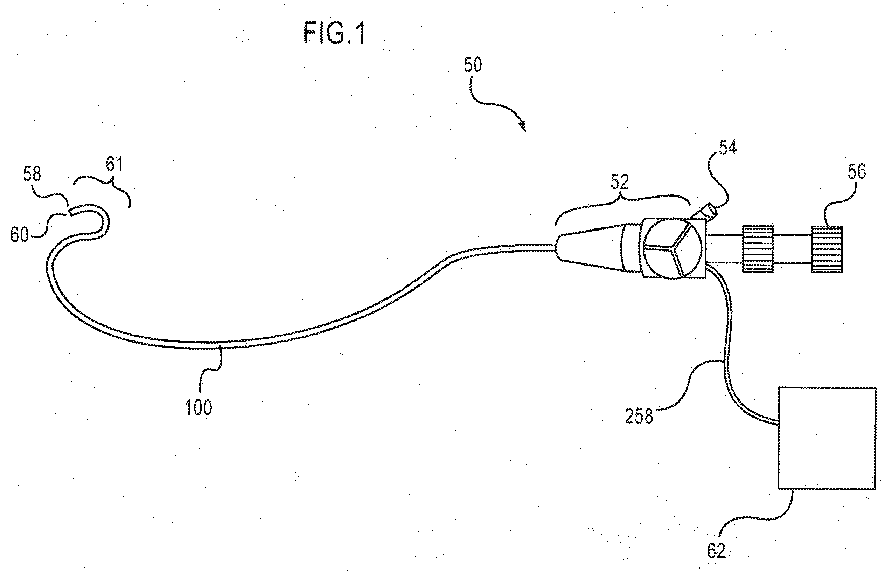

FIG. 1 shows an endoscope 50 with a proximal handle 52, an opening 54 for receiving a medical instrument, a viewing eyepiece 56, and an elongated shaft 100. At the tip 60 of the endoscope is a camera 58. The camera 58 can be a charge coupled device (CCD) or other type of image sensor useful for capturing images and/or full-motion video images in digital (or some other) format and then transmitting those images as electrical signals from the camera 58 back through one or more wires 258 extending within the shaft of the endoscope to a display unit 62. The tip 60 of the endoscope also may contain one or more light emitting diodes (LEDs) for illuminating internal body pathways and surgical sites inside a patient such as a human or other mammal. Fiber optics can also be used for illumination, although it can add stiffness and potentially take up more cross-sectional area. The elongated shaft 100 extends distally from the proximal handle 52. The terms proximal and distal require a point of reference. In this application, the point of reference is the perspective of the user. Therefore, the term proximal will always refer to an area closest to the user, whereas distal will always refer to an area away from the user.

The endoscope 50 can be any of a variety of types of scopes used in any of a variety of medical procedures. For example, the endoscope 50 can be a ureteroscope, a colonoscope, a hysteroscope, a uteroscope, bronchoscope, or a cystoscope.

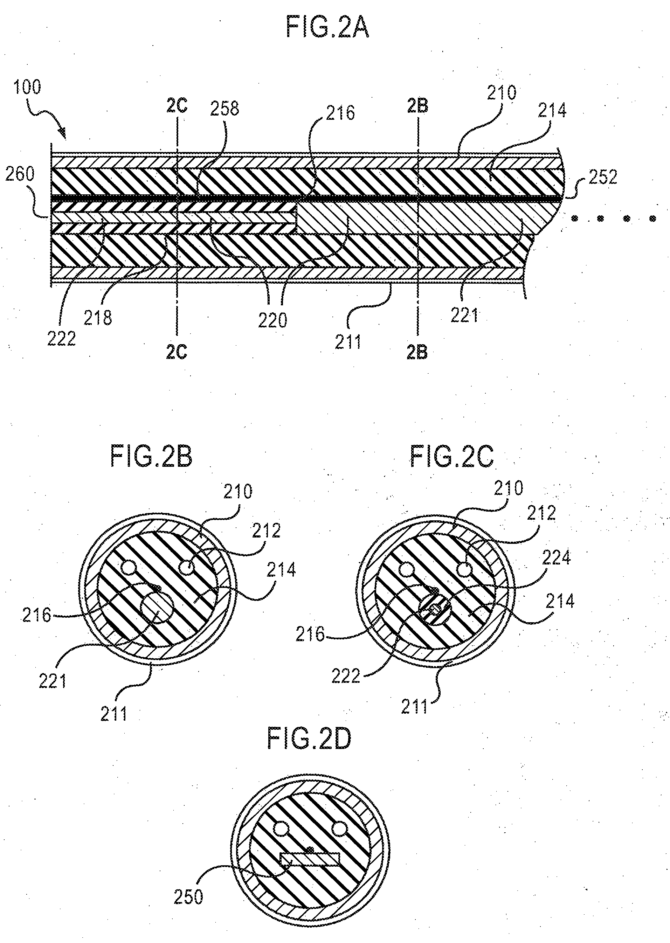

FIG. 2A shows a portion the elongated shaft 100. The right side 252 of the elongated shaft 100 shown in FIG. 2A extends distally from the proximal handle 52. The left side 260 of the elongated shaft shown in FIG. 2A can be located at a distal portion 61 of the medical device 50, or it can be the distal tip 60 of the medical device 50. FIG. 2B is a cross-section of the shaft at line 2B-2B of FIG. 2A. FIG. 2C is a cross-section of the shaft at line 2C-2C of FIG. 2A. All relative descriptions herein such as top, bottom, left, right, up, and down are with reference to the figures, and thus should not be construed in a limiting sense.

As shown in FIGS. 2A, 2B, and 2C, at least a portion of the shaft includes an inner member 214, a core member 220, and an outer member 210. The inner member can be made of a polymer, which can include polyethylene, polyvinyl chloride (PVC), polyurethane, teflon, polypropylene, nylon, polyether block amide sold under the trade name PEBAX, silicone, co-polymers, and other polymers. The inner member defines a first lumen 212 for receiving a medical instrument. The inner member may include one or more first lumens 212 for receiving a variety of instruments such as forceps, catheters, fiber optics, and other instruments. The inner member 214 also defines a second lumen 216 for receiving one or more wires 258 for connecting the camera 58 at a distal portion of the endoscope, for example, near or at the tip of the endoscope 50, to a viewing eyepiece 56 or display unit 62.

The inner member 214 also defines a third lumen 218. A core member 220 extends through the third lumen 218. The core member imparts column strength to the at least a portion of the elongated shaft, allowing the shaft to resist buckling as it is pushed through body pathways. The core member can occupy the space inside the endoscope that was traditionally occupied by imaging fiber optics, which have been replaced in the endoscope with the one or more camera wires 258.

The core member 220 can include a number of different configurations depending on the requirements of the endoscope. For example, different portions of the core member can be comprised of different materials with varying degrees of flexibility. Alternatively, the core member can be comprised of the same material with different cross sections, for example, circular or rectangular, with varying diameter or thickness. A combination of both material and cross section can be used.

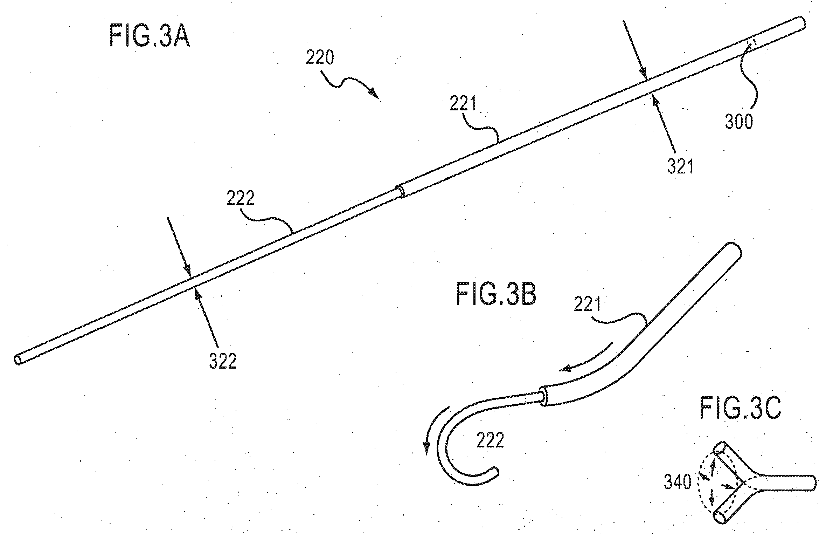

In one embodiment according to the invention, the core member 220 includes a proximal portion 221 and a distal portion 222 coupled to the proximal portion. As shown in FIG. 3A, the core member 220 can have a circular cross section 300. The proximal portion 221 can have a first diameter 321 and the distal portion 222 can have a second diameter 322. The first diameter is larger than the second diameter, imparting to the core member more flexibility over the distal portion 222 than the proximal portion 221, as shown in FIG. 3B. The circular core member can be used to impart 360 degrees of deflection 340 of the core member 220, as shown in FIG. 3C. In a further embodiment, the first diameter 321 of the proximal portion 221 is between 0.5 and 1.0 millimeters, and the second diameter 322 of the distal portion 222 is between 0.1 and 0.3 millimeters.

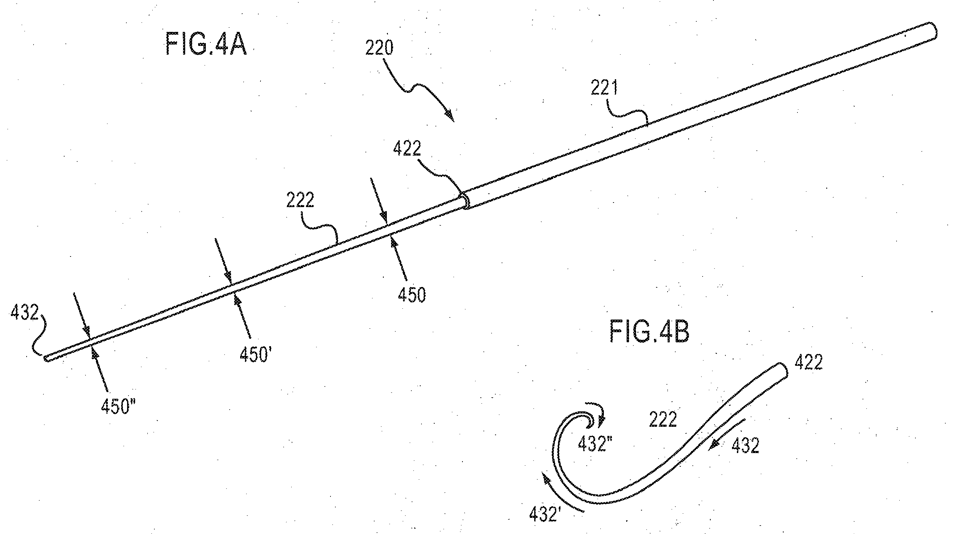

In another embodiment shown in FIG. 4A, the distal portion 222 has a proximal end 422 coupled to the proximal portion 221, and a distal end 432. The distal portion has a diameter that tapers 450, 450', and 450" from the proximal end, where the diameter is largest, to the distal end, where the diameter is smallest. In this way, the distal portion 221 is progressively more flexible 432, 432', and 432" toward the distal end 422 than at the proximal end 422, as shown in FIG. 4B.

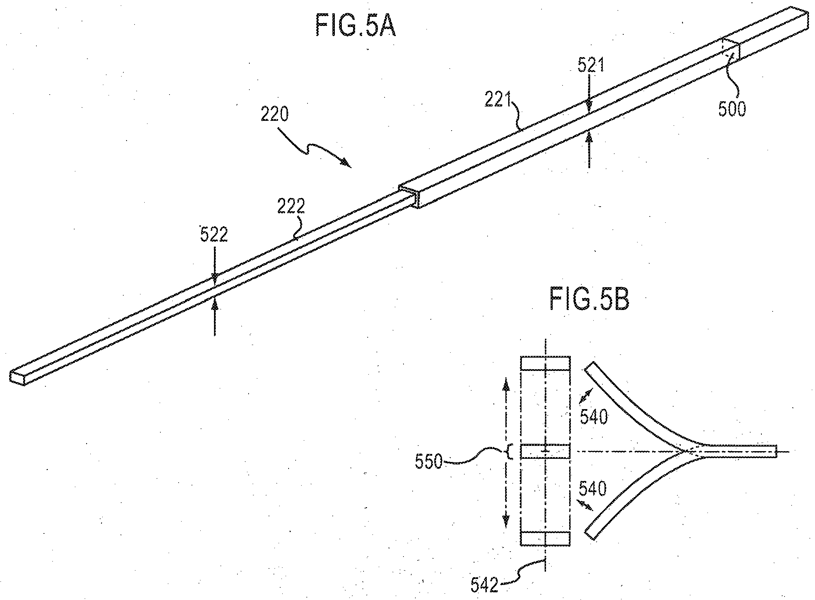

In one embodiment shown in FIG. 5A, the core member 220 has a rectangular cross section 500. The proximal portion can have a first thickness 521 and the distal portion can have a second thickness 522. The first thickness 521 is larger than the second thickness 522. The rectangular core member can be used to promote deflection 540 of the core member 220 in a single plane 542, with the shorter side of the core member 550 in the bending plane, as shown in FIG. 5B.

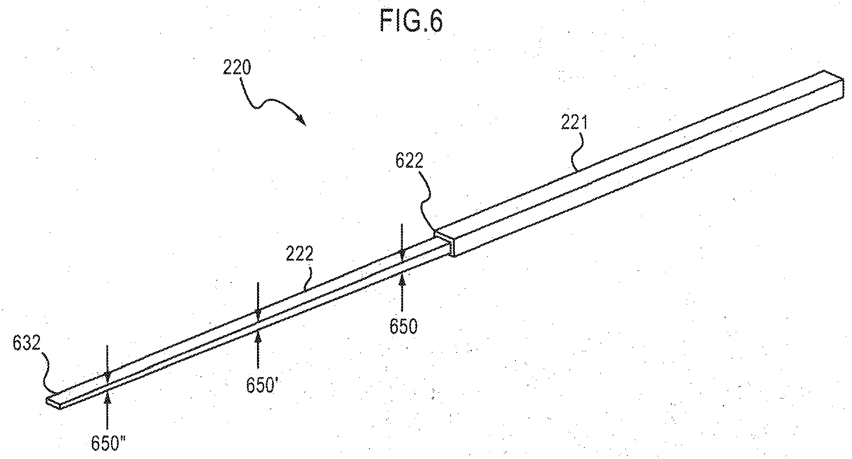

In another embodiment shown in FIG. 6, the distal portion 222 has a proximal end 622 coupled to the proximal portion 221, and a distal end 632. The distal portion has a thickness that tapers 650, 650', and 650" from the proximal end, where the thickness is largest, to the distal end, where the thickness is smallest. In this way, the distal portion 221 is progressively more flexible toward the distal end 632 than at the proximal end 622.

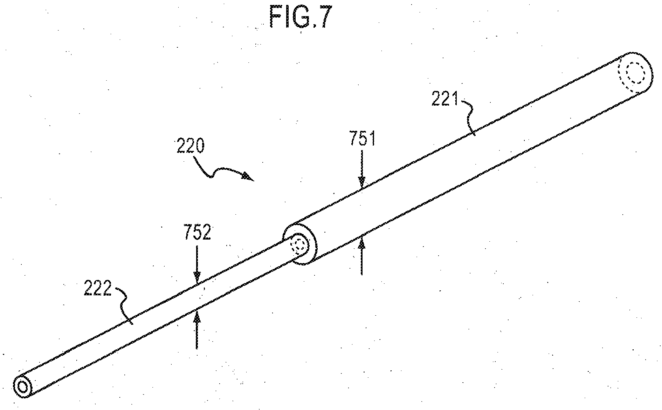

In one embodiment shown in FIG. 7, the core member 220 can be a hollow tube with a proximal portion 221 and a distal portion 222 coupled to the proximal portion. The proximal portion can have a first diameter 751 and the distal portion can have a second diameter 752. The first diameter is larger than the second diameter, imparting to the core member more flexibility over the distal portion than the proximal portion. Instruments or other surgical devices can be disposed in the hollow core member.

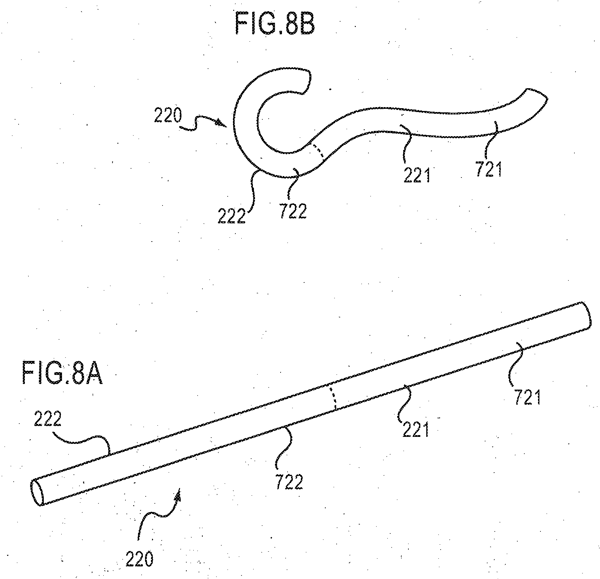

In one embodiment shown in FIGS. 8A and 8B, the core member 220 can include a proximal portion 221 comprised of a first material 721 and a distal portion 222 coupled to the proximal portion 221 and comprised of a second material 722. The first material can be less flexible than the second material, imparting more flexibility to the distal portion as shown in FIG. 8B. The first material can be stainless steel, and the second material can be a shape memory alloy such as a Nitinol™ (an alloy of nickel and titanium). Examples of other materials include tungsten alloys, and other more malleable alloys, including gold, platinum, palladium, rhodium, etc. The class of alloys known as superelastic alloys can also be used, including titanium. Non-metal materials with varying amounts of flexibility, for example, composite materials, could also be used.

In any of FIGS. 2A, 2B, and 2C, the outer member 210 can be covered with a lubricious material 211 that makes it "slippery" on its outer surface. One such material 211 is known as Teflon, which is a trademark used for a waxy, opaque material called polytetrafluoroethylene. In one embodiment, the outer member 210 comprises a plastic or a polymer, which can include polyethylene, polyvinyl chloride (PVC), PEBAX, silicone, co-polymers, and other polymers. The outer member can be heat shrunk over the inner member 214 into tight engagement with the inner member. The outer member can be secured to the inner member with an adhesive applied on the outer surface of the inner member or at various affixation points on the outer surface of the inner member. The adhesive can be a thermo-plastic adhesive that softens at the temperature necessary to heat shrink the outer member.

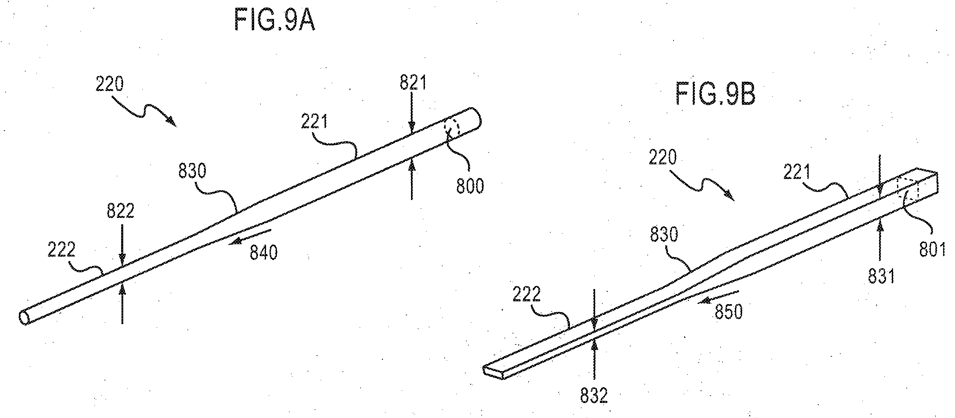

In one embodiment shown in FIG. 9A, the core member 220 includes a proximal portion 221, a distal portion 222, and a transitional portion 830 coupling the proximal portion to the distal portion. The core member 220 can have a circular cross section 800, and the proximal portion can have a first diameter 821 and the distal portion can have a second diameter 822, the first diameter being larger than the second diameter. The transitional portion 830 has a tapered diameter that reduces 840 from the first diameter 821 to second diameter 822. In another embodiment shown in FIG. 9B, the core member 220 can have a rectangular cross section 801, and the proximal portion can have a first thickness 831 and the distal portion can have a second thickness 832, the first thickness being larger than the second thickness. The transitional portion 830 can have a tapered thickness that reduces 850 from the first thickness to the second thickness.

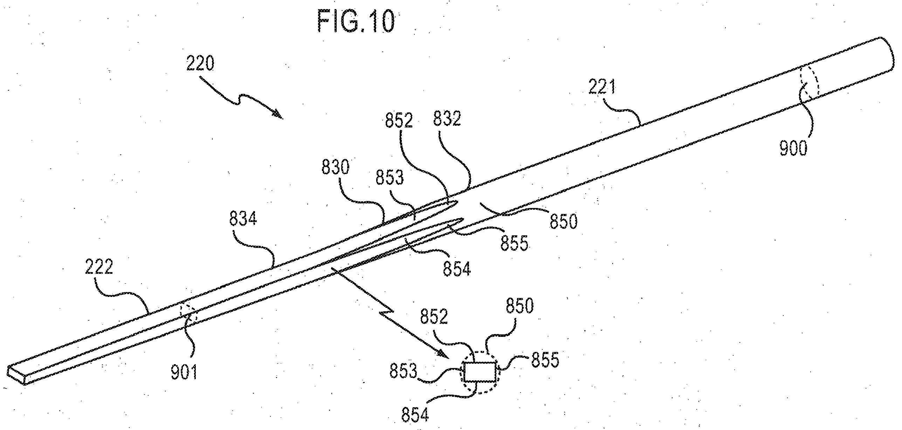

In another embodiment shown in FIG. 10, the proximal portion 221 can have a circular cross section 900 and the distal portion 222 can have a rectangular cross section 901. The transitional portion 830 can have a cross section that progressively changes from circular where the transitional portion is coupled to the proximal portion at 832 to rectangular where the transitional portion is coupled to the distal portion at 834. In another embodiment, the transitional portion includes an outer surface 850, wherein the progressive change comprises a flattening on four sides 852, 853, 854, and 855 of the outer surface 850, starting from the circular portion of the transitional portion and ending at the rectangular portion of the transitional portion, such that the transitional portion cross-section progressively changes from a rectangular to circular.

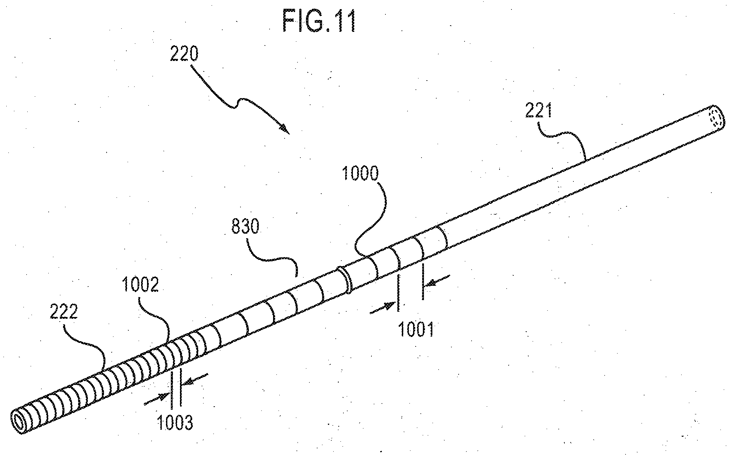

In one embodiment shown in FIG. 11, the core member 220 is a hollow tube and includes a proximal portion 221, a distal portion 222, and a transitional portion 830 coupling the proximal and distal portions. The transitional portion has a helically cut slot 1000 with a pitch 1001 defined as the distance between adjacent slots. The distal portion has a helically cut slot 1002 with a pitch 1003 defined as the distance between the adjacent slots. The pitch of the transitional portion 1001 can be greater than the pitch of the distal portion 1003, resulting in the transitional portion being less flexible than the distal portion.

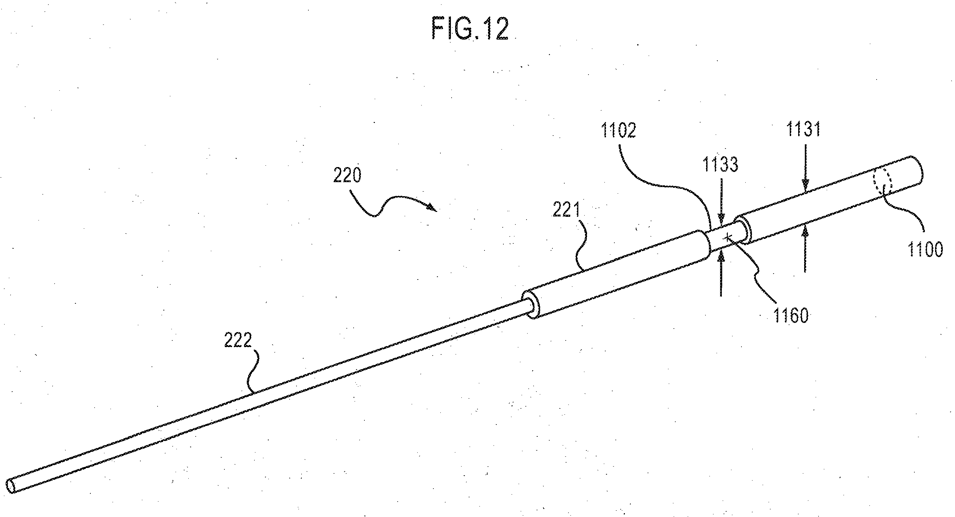

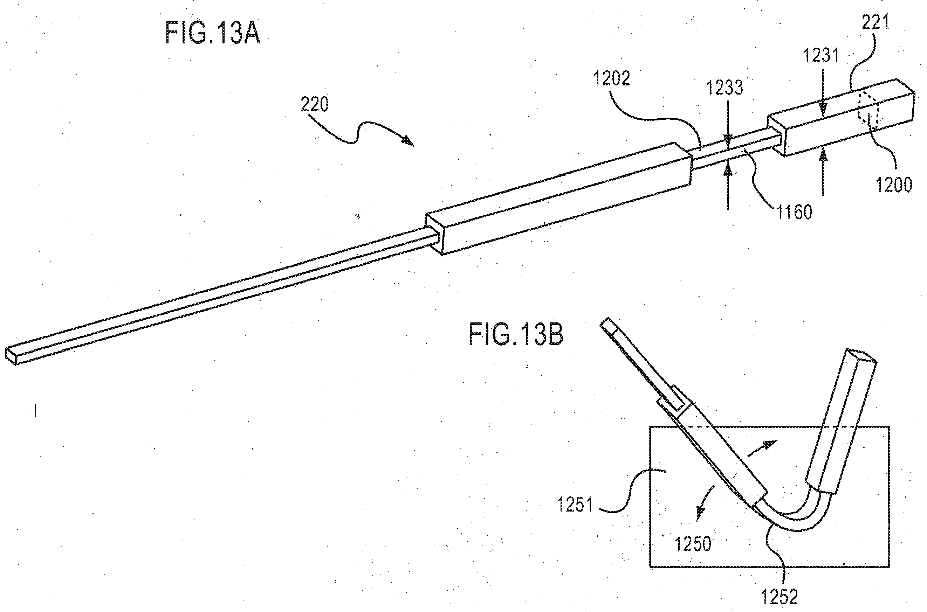

In one embodiment shown in FIG. 12, the core member 220 has a circular cross section 1100, and the proximal portion has a first diameter 1131 and includes a deflection portion 1102 with a second diameter 1133 that is smaller than the first diameter. The deflection portion 1102 promotes deflection of the core member along the deflection portion. In another embodiment shown in FIG. 13A, the core member 220 has a rectangular cross section 1200 and the proximal portion 221 has a first thickness 1231 and includes deflection portion 1202 with a second thickness 1233 that is smaller than the first thickness. At shown in FIG. 13B, the deflection portion 1202 promotes deflection 1250 in a single plane 1251, with the shorter side 1252 of the core member in the deflection plane. In some embodiments, for example in a ureteroscope, he center point 1160 of the deflection portion can be between 8 and 15 centimeters from the distal end of the elongated shaft 100, although other dimensional ranges may be appropriate for other medical applications. In another embodiment, the distal portion has a deflection portion with a smaller diameter or thickness than the distal portion.

The assembling procedure for the endoscope 50 and elongated shaft 100 can include extruding the inner member 214 inside the lumen of the outer member 210. Alternatively, the outer member 210 can be heat-shrunk over the inner member 214. The first lumen 212, second lumen 216, and third lumen 218 can be built into the inner member. The core member 220 can be inserted into the third lumen and locked into place with glue or other type of adhesive. The core member can include the proximal portion 221 and distal portion 222 constructed with varying flexibility and column strength as described above to meet the design needs for the endoscope. The space between the reduced diameter or thickness portion of the core member can be filled with a soft material 224.

An endoscope according to the invention has a variety of advantages over known structures. For example, an endoscope according to the invention can be less expensive to manufacture than known endoscopes. Another advantage is that use of a central core member can reduce the overall diameter of the shaft of the endoscope as compared to known endoscopes, making the inventive endoscope less invasive.

While certain embodiments according to the invention are shown and described, other embodiments are within the scope of this disclosure and are considered to be part hereof. The invention is not to be limited just to certain embodiments shown and/or described.

## Claims

1. An endoscope, comprising:
a proximal handle (52); and
an elongated shaft (100) extending distally from the proximal handle (52), at least a portion of the shaft comprising:
an inner member (214) defining a first lumen (212) for receiving an instrument, a second lumen (216) for receiving one or more wires (258) for connecting a camera (58) at a distal portion of the elongated shaft (100) to a display unit (62), and a third lumen (218);
a core member (220) extending through the third lumen (218) of the inner member (214), the core member (220) being flexible and providing column strength to the at least a portion of the shaft (100), and the core member (220) being locked in place with glue or other types of adhesive with respect to the third lumen (218); and
an outer member (210) disposed over the inner member (214), the outer member (210) defining a lumen through which the inner member (214) extends.

2. The endoscope of claim 1 wherein the core member (220) further comprises proximal portion (221) and a distal portion (222) coupled to the proximal portion.

3. The endoscope of claim 2 wherein the core member (220) has a circular cross section and the proximal portion (221) has a first diameter and the distal portion (222) has a second diameter, the first diameter being larger than the second diameter.

4. The endoscope of claim 2 wherein the core member (220) has a circular cross section and at least a portion of the distal portion (222) is tapered.

5. The endoscope of claim 2 wherein the core member (220) has a rectangular cross section and the proximal portion (221) has a first thickness and the distal portion (222) has a second thickness, the first thickness being larger than the second thickness.

6. The endoscope of claim 2 wherein the core member (220) has a rectangular cross section and at least a portion of the distal portion (222) is tapered.

7. The endoscope of claim 2 wherein the core member (220) is a hollow tube and the proximal portion (221) has a first diameter and the distal portion (222) has a second diameter, the first diameter being larger than the second diameter.

8. The endoscope of claim 2 wherein the proximal portion (221) comprises a first material and the distal portion (222) comprises a second material, the first material being less flexible than the second material.

9. The endoscope of claim 2 wherein the core member (220) further comprises a transitional portion (830) coupling the proximal portion to the distal portion.

10. The endoscope of claim 9 wherein the core member (220) has a circular cross section and the proximal portion (221) has a first diameter and the distal portion (222) has a second diameter, the first diameter being larger than the second diameter, and the transitional portion (830) is tapered to transition from the first diameter to the second diameter.

11. The endoscope of claim 9 wherein the core member (220) has a rectangular cross section and the proximal portion (221) has a first thickness and the distal portion (222) has a second thickness, the first thickness being larger than the second thickness, and the transitional portion (830) is tapered to transition from the first thickness to the second thickness.

12. The endoscope of claim 9 wherein the proximal portion (221) has a circular cross section and the distal portion (222) has a rectangular cross section, the cross section of the transitional portion (830) progressively changing from circular to rectangular.

13. The endoscope of claim 2 wherein the core member (220) has a circular cross section and the proximal portion (221) has a first diameter and the proximal portion further comprises: a deflection portion (1102) having a second diameter that is smaller than the first diameter to promote deflection of the elongated shaft along the deflection portion.

14. The endoscope of claim 2 wherein the core member (220) has a rectangular cross section and the proximal portion (221) has a first thickness and the proximal portion further comprises: a deflection portion (1233) having a second thickness that is smaller than the first thickness to promote deflection of the elongated shaft along the deflection portion.

15. The endoscope of claim 2 wherein the distal portion (222) further comprises: a deflection portion having a cross sectional area that is smaller than the distal portion cross sectional area to promote deflection of the elongated shaft along the deflection portion.

## Patentansprüche

1. Endoskop, welches aufweist:
einen proximalen Handgriff (52); und
eine längliche Welle (100), die sich distal von dem proximalen Handgriff (52) erstreckt, wobei zumindest ein Bereich der Welle aufweist:
in inneres Teil (214), das ein erstes Lumen (212) für die Aufnahme eines Instruments, ein zweites Lumen (216) für die Aufnahme von einem oder mehreren Drähten (258) zur Verbindung einer Kamera (58) an einem distalen Bereich der länglichen Welle (100) mit einer Anzeigeeinheit (62), und ein drittes Lumen (218) definiert;
ein Kernelement (220), das sich durch das dritte Lumen (218) des inneren Teils (214) erstreckt, wobei das Kernelement (220) flexibel ist und zumindest einem Bereich der Welle (100) Knickfestigkeit verleiht, und das Kernelement (220) mit Leim oder anderen Typen von Klebstoff in seiner Lage mit Bezug auf das dritte Lumen (218) fixiert ist; und
ein äußeres Teil (210), das über dem inneren Teil (214) angeordnet ist, wobei das äußere Teil (210) ein Lumen definiert, durch das sich das innere Teil (214) erstreckt.

2. Endoskop nach Anspruch 1, bei dem das Kernelement (220) weiterhin einen proximalen Bereich (221) und einen distalen Bereich (222), der mit dem proximalen Bereich gekoppelt ist, aufweist.

3. Endoskop nach Anspruch 2, bei dem das Kernelement (220) einen kreisförmigen Querschnitt hat und der proximale Bereich (221) einen ersten Durchmesser hat und der distale Bereich (222) einen zweiten Durchmesser hat, wobei der erste Durchmesser größer als der zweite Durchmesser ist.

4. Endoskop nach Anspruch 2, bei dem das Kernelement (220) einen kreisförmigen Querschnitt hat und zumindest ein Bereich des distalen Bereichs (222) konisch ist.

5. Endoskop nach Anspruch 2, bei dem das Kernelement (220) einen rechteckigen Querschnitt hat und der proximale Bereich (221) eine erste Dicke hat und der distale Bereich (222) eine zweite Dicke hat, wobei die erste Dicke größer als die zweite Dicke ist.

6. Endoskop nach Anspruch 2, bei dem das Kernelement (220) einen rechteckigen Querschnitt hat und zumindest ein Bereich des distalen Bereichs (222) konisch ist.

7. Endoskop nach Anspruch 2, bei dem das Kernelement (220) ein hohles Rohr ist und der proximale Bereich (221) einen ersten Durchmesser hat und der distale Bereich (222) einen zweiten Durchmesser hat, wobei der erste Durchmesser größer als der zweite Durchmesser ist.

8. Endoskop nach Anspruch 2, bei dem der proximale Bereich (221) ein erstes Material aufweist und der distale Bereich (222) ein zweites Material aufweist, wobei das erste Material weniger flexibel als das zweite Material ist.

9. Endoskop nach Anspruch 2, bei dem das Kernelement (220) weiterhin einen Übergangsbereich (830) aufweist, der den proximalen Bereich mit dem distalen Bereich koppelt.

10. Endoskop nach Anspruch 9, bei dem das Kernelement (220) einen kreisförmigen Querschnitt hat und der proximale Bereich (221) einen ersten Durchmesser hat und der distale Bereich (222) einen zweiten Durchmesser hat, wobei der erste Durchmesser größer als der zweite Durchmesser ist, und der Übergangsbereich (830) konisch ist für den Übergang von dem ersten Durchmesser zu dem zweiten Durchmesser.

11. Endoskop nach Anspruch 9, bei dem das Kernelement (220) einen rechteckigen Querschnitt hat und der proximale Bereich (221) eine erste Dicke hat und der distale Bereich (222) eine zweite Dicke hat, wobei die erste Dicke größer als die zweite Dicke ist, und der Übergangsbereich (830) konisch ist für den Übergang von der ersten Dicke zu der zweiten Dicke.

12. Endoskop nach Anspruch 9, bei dem der proximale Bereich (221) einen kreisförmigen Querschnitt hat und der distale Bereich (222) einen rechteckigen Querschnitt hat, wobei der Querschnitt des Übergangsbereichs (830) sich fortschreitend von kreisförmig in rechteckig ändert.

13. Endoskop nach Anspruch 2, bei dem das Kernelement (220) einen kreisförmigen Querschnitt hat und der proximale Bereich (221) einen ersten Durchmesser hat und der proximale Bereich weiterhin aufweist: einen Biegungsbereich (1102) mit einem zweiten Durchmesser, der kleiner als der erste Durchmesser ist, um eine Biegung der länglichen Welle entlang des Biegungsbereichs zu fördern.

14. Endoskop nach Anspruch 2, bei dem das Kernelement (220) einen rechteckigen Querschnitt hat und der proximale Bereich (221) eine erste Dicke hat und der proximale Bereich weiterhin aufweist: einen Biegungsbereich (1233) mit einer zweiten Dicke, die kleiner als die erste Dicke ist, um eine Biegung der länglichen Welle entlang des Biegungsbereichs zu fördern.

15. Endoskop nach Anspruch 2, bei dem der distale Bereich (222) weiterhin aufweist: einen Biegebereich mit einer Querschnittsfläche, die kleiner als die Querschnittsfläche des distalen Bereichs ist, um eine Biegung der länglichen Welle entlang des Biegungsbereichs zu fördern.

## Revendications

1. Endoscope, comprenant:
un manche proximal (52) ; et
un arbre allongé (100) qui s'étend de façon distale depuis le manche proximal (52), au moins une partie de l'arbre comprenant :
un élément interne (214) qui définit une première lumière (212) pour recevoir un instrument, une seconde lumière (216) pour recevoir un ou plusieurs fil(s) (258) pour connecter une caméra (58) au niveau d'une partie distale de l'arbre allongé (100) à une unité d'affichage (62), et une troisième lumière (218) ;
un élément d'âme (220) qui s'étend au travers de la troisième lumière (218) de l'élément interne (214), l'élément d'âme (220) étant flexible et assurant une résistance mécanique colonnaire pour l'au moins une partie de l'arbre (100), et l'élément d'âme (220) étant verrouillé en place à l'aide de colle ou d'autres types d'adhésif par rapport à la troisième lumière (218) ; et
un élément externe (210) disposé au dessus de l'élément interne (214), l'élément externe (210) définissant une lumière au travers de laquelle l'élément interne (214) s'étend.

2. Endoscope selon la revendication 1, dans lequel l'élément d'âme (220) comprend en outre une partie proximale (221) et une partie distale (222) couplée à la partie proximale.

3. Endoscope selon la revendication 2, dans lequel l'élément d'âme (220) présente une section en coupe circulaire et la partie proximale (221) présente un premier diamètre et la partie distale (222) présente un second diamètre, le premier diamètre étant plus grand que le second diamètre.

4. Endoscope selon la revendication 2, dans lequel l'élément d'âme (220) présente une section en coupe circulaire et au moins une partie de la partie distale (222) est conique.

5. Endoscope selon la revendication 2, dans lequel l'élément d'âme (220) présente une section en coupe rectangulaire et la partie proximale (221) présente une première épaisseur et la partie distale (222) présente une seconde épaisseur, la première épaisseur étant plus grande que la seconde épaisseur.

6. Endoscope selon la revendication 2, dans lequel l'élément d'âme (220) présente une section en coupe rectangulaire et au moins une partie de la partie distale (222) est conique.

7. Endoscope selon la revendication 2, dans lequel l'élément d'âme (220) est un tube creux et la partie proximale (221) présente un premier diamètre et la partie distale (222) présente un second diamètre, le premier diamètre étant plus grand que le second diamètre.

8. Endoscope selon la revendication 2, dans lequel la partie proximale (221) comprend un premier matériau et la partie distale (222) comprend un second matériau, le premier matériau étant moins flexible que le second matériau.

9. Endoscope selon la revendication 2, dans lequel l'élément d'âme (220) comprend en outre une partie de transition (830) qui couple la partie proximale à la partie distale.

10. Endoscope selon la revendication 9, dans lequel l'élément d'âme (220) présente une section en coupe circulaire et la partie proximale (221) présente un premier diamètre et la partie distale (222) présente un second diamètre, le premier diamètre étant plus grand que le second diamètre, et la partie de transition (830) est conique afin de réaliser une transition depuis le premier diamètre jusqu'au second diamètre.

11. Endoscope selon la revendication 9, dans lequel l'élément d'âme (220) présente une section en coupe rectangulaire et la partie proximale (221) présente une première épaisseur et la partie distale (222) présente une seconde épaisseur, la première épaisseur étant plus grande que la seconde épaisseur, et la partie de transition (830) est conique afin de réaliser une transition depuis la première épaisseur jusqu'à la seconde épaisseur.

12. Endoscope selon la revendication 9, dans lequel la partie proximale (221) présente une section en coupe circulaire et la partie distale (222) présente une section en coupe rectangulaire, la section en coupe de la partie de transition (830) variant de façon progressive de circulaire à rectangulaire.

13. Endoscope selon la revendication 2, dans lequel l'élément d'âme (220) présente une section en coupe circulaire et la partie proximale (221) présente un premier diamètre et la partie proximale comprend en outre : une partie de fléchissement (1102) présentant un second diamètre qui est plus petit que le premier diamètre afin de favoriser un fléchissement de l'arbre allongé suivant la partie de fléchissement.

14. Endoscope selon la revendication 2, dans lequel l'élément d'âme (220) présente une section en coupe rectangulaire et la partie proximale (221) présente une première épaisseur et la partie proximale comprend en outre : une partie de fléchissement (1233) présentant une seconde épaisseur qui est plus petite que la première épaisseur afin de favoriser un fléchissement de l'arbre allongé suivant la partie de fléchissement.

15. Endoscope selon la revendication 2, dans lequel la partie distale (222) comprend en outre : une partie de fléchissement présentant une aire en coupe qui est plus petite que l'aire en coupe de la partie distale afin de favoriser un fléchissement de l'arbre allongé suivant la partie de fléchissement.
